(19) Europäisches Patentamt
European Patent Office
Office européen des brevets

(11) **EP 1 857 950 A2**

(12) **EUROPEAN PATENT APPLICATION**

(43) Date of publication:
**21.11.2007 Bulletin 2007/47**

(51) Int Cl.:
***G06F 19/00*** (2006.01)

(21) Application number: **07107471.0**

(22) Date of filing: **03.05.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK YU**

(30) Priority: **18.05.2006 CN 200610082709**

(71) Applicant: **Taiwan Chest Disease Association 333 Taoyuan County (TW)**

(72) Inventors:
• **Juang, Jer-Nan**
  **Taoyuan County 333 (TW)**
• **Kuo, Han-Pin**
  **Taoyuan County 333 (TW)**
• **Liu, Wen-Te**
  **Taoyuan County 333 (TW)**
• **Hung, Shu-Hui**
  **Taoyuan County 333 (TW)**

(74) Representative: **Viering, Jentschura & Partner Grillparzerstrasse 14 81675 München (DE)**

(54) **Method and system for remotely analysing and managing the health state of a patient**

(57)    Method, Internet grid platform, and mobile phone for analyzing and managing for telehealth are disclosed. The method discloses: receiving status data from a patient; obtaining position data of the patient; accessing environment data according to the position data; and analyzing the status data according to the environment data for obtaining analyzed data.

**Fig. 1**

EP 1 857 950 A2

**Description**

**Background of the Invention**

**1. Field of the Invention**

The present invention relates to a method, Internet grid platform, and mobile phone for analyzing and managing for telehealth.

**2. Description of the Prior Art**

When people get sick, usually, their first response is seeking for medical help. In general, their disease will be diagnosed by a doctor, and then they are treated properly according to the severity of the disease.

For most chronic diseases such as asthma, diabetes or hypertension, patients receive medical suggestion in the clinical return visit periodically (such as every four to twelve weeks). The severity of these diseases may change before they see the doctor. However, the periodically outpatient clinical services or the long-period prescription can only provide passive defenses, instead of active controlling by doctors or medical hospital for any possible serious situation of the patient.

In addition, periodically outpatient services can't distribute the medical sources efficiently. Some chronic diseases, especially "asthma" was largely influenced by the environmental factors in the disease control. These factors including change of atmospheric temperature, weather moisture and the amount of pollutant may cause acute exacerbation of asthma even respiratory distress. Though the doctors or the hospitals know very well about this situation, they just cannot provide any aids immediately for those patients when they need help.

**Summary of the Invention**

One aspect of the present invention is to solve the above-mentioned problem. Therefore, the present invention provides method, Internet platform, and mobile phone using the same of analyzing and managing for a health state of a patient. One object of the invention is to allow the patient uploading his/her subjective symptoms and objective measuring to the Internet platform, by that the doctors or hospitals can effectively monitor the patient for his/her conditions through a record database.

Another object of the invention is that the doctors or the hospitals may provide aggressive health consultation for those patients who may be affected seriously by environment, and further to allow the patient to have self assessments and cares.

In short, a user (or a patient) may enter and complete a physical state information survey by sending message (s) via a mobile phone to an Internet platform, alternatively by registering the Internet platform via wired or wireless accessing to a webpage or the like.

The Internet platform of analyzing and managing for a health state of a patient according to this invention comprises a processor and a memory element having software stored therein. The processor executes the software stored in the memory element to perform:

A) receiving at least one physical state information of the patient;
B) determining present location information of the patient;
C) obtaining environment information associated with the present location information; and
D) analyzing the physical state information according to the environment information to generate at least one analyzed information.

If the user (or the patient) sends the message(s)of the physical state information survey through the mobile phone, the Internet platform then may obtain the location information of the user of the mobile phone based on a cellular station. Alternatively, the user (or the patient) may complete the physical state information survey and enter his/her location by using a computer connection with Internet.

The Internet platform of this invention further comprises an environment database, a health standard database, and a record database. The record database is adapted to store all physical state information received from the user (or the patient). The environment database is adapted to store the environment information, such as temperature data, moisture data, and/or suspended particulates data. The health standard database is adapted to store health standard data (such as a formula of health standard) for assessing the patient. The Internet platform is provided for the processor executing the software to perform:

setting an environment standard; and

comparing the updated environment information with the environment standard to automatically send a message associated with a varied environment information for informing the patient to enter his/her physical state information.

Accordingly, all the patient's physical state information in the record database can be calculated to find out the possible factor that may make the patient more serious.

The Internet platform according to this invention may obtain environment information responsive to the location information of the patient, wherein the environment information is stored in the environment database and can be real-time updated. According to the obtained environment information, the Internet platform may send a message associated with varied environment information to ask the patient entering his/her physical state information. The Internet platform analyzes the physical state information, and then generates analyzed information, such as physical state information of the patient to monitor and manage the health state of the patient.

**Brief Description of the Drawings**

Fig. 1 illustrates an architecture diagram of an Internet platform for analyzing and managing for a health state of a patient in accordance with an embodiment of the present invention.

Fig. 2 shows a flow diagram of a method of analyzing and managing for a health state of a patient in accordance with an embodiment of the present invention.

Fig. 3 shows a physical state information survey for a user to enter in accordance with an embodiment of the present invention.

Fig. 4 shows 18 standard levels of asthma.

Fig. 5A-5C shows exemples of analyzed information that is analyzed according to the physical state information provided by the patient.

Fig. 6 shows a flow diagram of a method of informing the patient about environment information in accordance with an embodiment of the present invention.

Fig. 7 illustrates a block diagram of a mobile phone for analyzing and managing for a health state of a patient in accordance with an embodiment of the present invention.

**Detailed Description of the Invention**

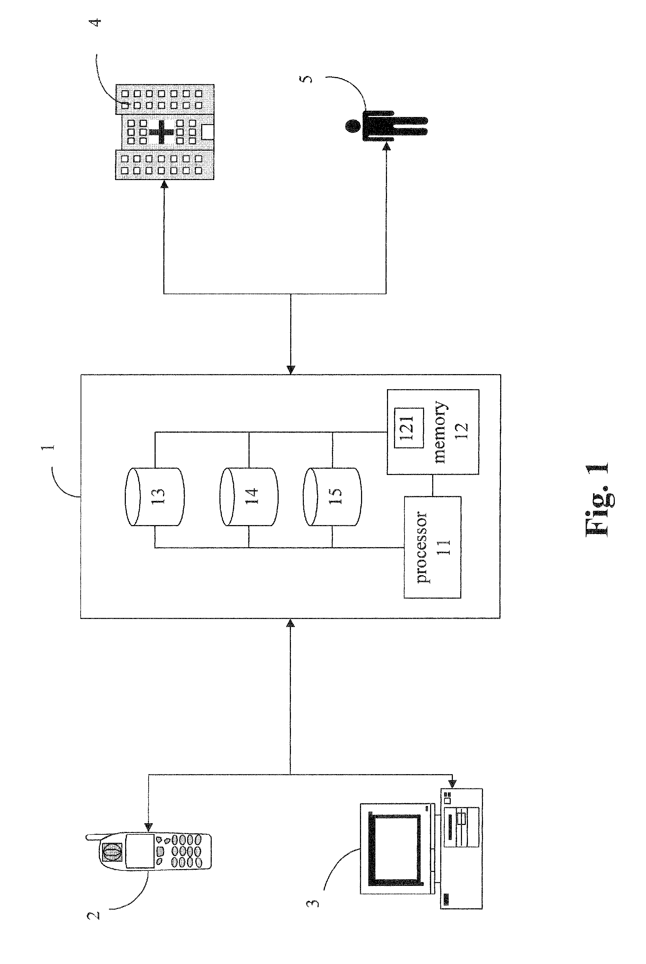

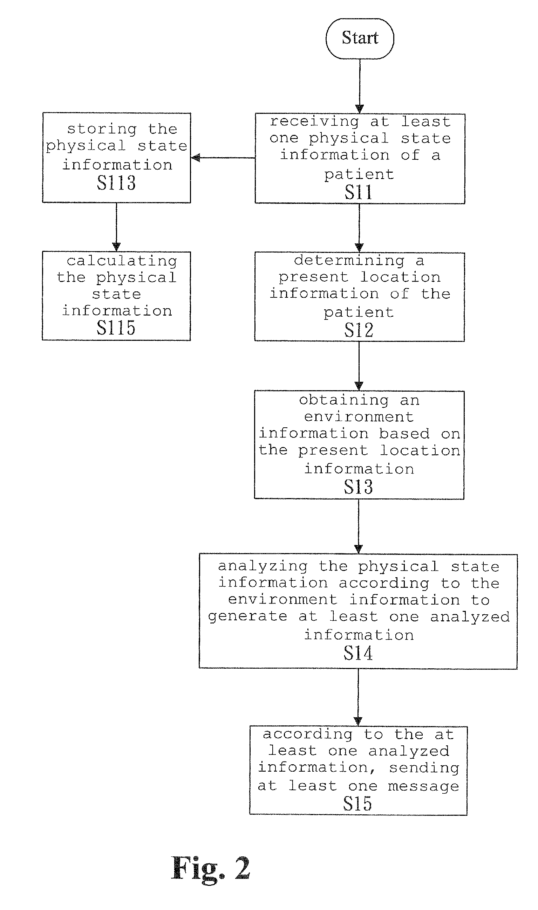

Please refer to Fig. 1 and 2. Fig. 1 illustrates an architecture diagram of an Internet platform for analyzing and managing for a health state of a patient in accordance with an embodiment of the present invention. Fig. 2 shows a flow diagram of a method of analyzing and managing for a health state of a patient in accordance with an embodiment of the present invention.

As shown in Fig. 1, Internet platform 1 of this invention comprises a processor 11, a memory element 12 having software 121 therein, an environment database 13, a health standard database 14, and a record database 15. The Internet platform 1 can be, for example, configured in a server (not shown).

The environment database 13 stores with environment information that comprises at least one of temperature data, moisture data, and suspended particulates data. And, the environment database 13 can update the environment information stored therein. The environment information can be updated according the national weather report center.

The health standard database 14 stores with health standard data that is accorded with the standards set by WHO or Global Initiative for Asthma (GINA).

The record database 15 can store with physical state information of the patient. In an embodiment of the present invention, the physical state information can be provided by the patient. For example, the physical state information can be provided by sending message from a mobile phone 2. Optionally, the physical state information can be provided by a computer 3 or the mobile phone 2 accessing the record database 15 via Internet. For example, the mobile phone 2 can store with application software for executing of providing physical state information by displaying a plurality of physical states on a display device to be chosen by the patient. The patient can enter and/or click the physical states for such choosing. Furthermore, the patient can use the mobile phone 2 to send a message containing the physical state information to the Internet platform 1. Preferably, the physical state information is further stored in the record database 15.

The processor 11 and the memory element 12 are electronically connected to each other so that the processor 11 can execute the software 121 contained in the memory element 12 to achieve for analyzing and managing for the health state of the patient, as shown in Fig. 2.

Please refer to Fig. 2 of the flow diagram of analyzing and managing for a health state of a patient in accordance with an embodiment of the present invention. After start, the process of the present invention comes to block S11:

receiving at least one physical state information of the patient. As described in above, the patient can use the mobile phone 2 or the computer 3 to enter his/her physical state information. And then, the mobile phone 2 or the computer 3 transmits the physical state information to the Internet platform 1.

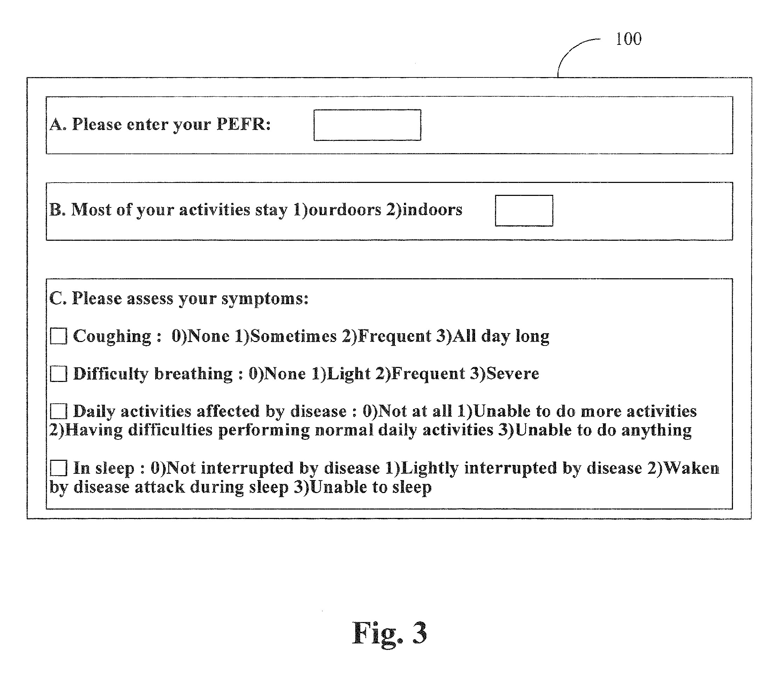

For example, the mobile phone 2 or the computer 3 can have a display device (not shown), referring to Fig. 3, for displaying a survey 100 to be entered the physical state information by the patient. The physical state information may include, but not limited to, sleeping condition data, breathing status level data, coughing status level data, activity status data, or peak expiratory flow rate (PEFR) data.

Then next, the process comes to block S12: determining present location information of the patient. Please see back to the Fig. 1. In an embodiment, the patient uses the mobile phone 2 to send the message containing the physical state information to the Internet platform 1. According to cell sites positioning, when the mobile phone 2 sends the message, one of the cell sites would be found for the mobile phone 2. Thus, the Internet platform 1 can obtain present location information of the mobile phone 2 of the patient based on the cell site.

Alternatively, in another embodiment, when the patient connects with Internet by using the computer 3 or the mobile phone 2 wirenessly or wirelessly, he/she can login the internet platform 1 and complete the entering of physical state information, as described in above. In this embodiment, the present location information of the patient can be entered manually for the Internet platform 1 to obtain the present location information. Optionally, the present location information of the patient can be registered previously. Thus, when the patient login the Internet platform 1, the location information can be shown for confirmation.

Then next, the process comes to block S13: obtaining environment information associated with the present location information. The environment database 13, as shown in Fig. 1, stores with environment information. The environment information may comprise at least one of temperature data, moisture data, or suspended particulates data. The environment information stored in the environment database 13 can be updated according to the National Weather Report Center. Therefore, the Internet platform 1 may obtain environment information associated with the present location information of the patient.

Next, the process of the present invention comes to block S14: analyzing the physical state information according to the environment information to generate at least one analyzed information.

It should be understood that the above steps are not used to limited the present invention. For example, the Internet platform 1 may first obtain the present location information of the patient (S12) and the associated environment information (S13) for sending the message to the mobile phone 2 to inform the patient to enter the physical state information (S11) for analyzing the physical state information and generating the analyzed information (S14).

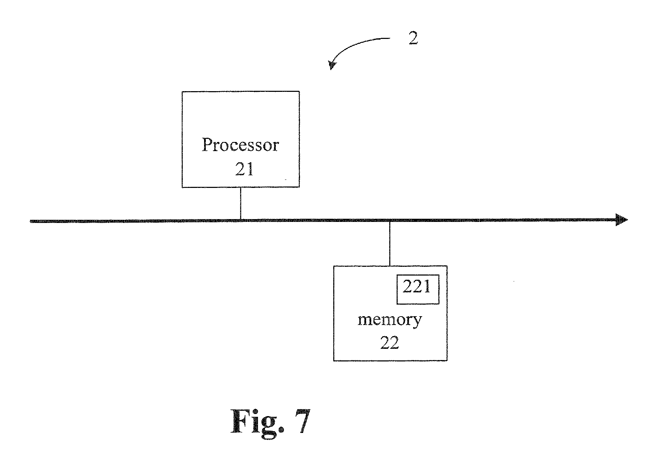

The survey 100 shown in Fig. 3 can be used for the patient to enter the physical state information. And the physical state information can be calculated by a formula according to the standards set by WHO or Global Initiative for Asthma (GINA) for analyzing thereof. The formula may be stored in the memory element 12 of the Internet platform 1 or memory element 22 of the mobile phone 2 (shown in Fig. 7). The calculation will be described in below.

The physical state information is compared with the health standard data, or set the physical state information into the formula for generating at least one analyzed information. For example, the doctor can set a best PEFR for the patient as a standard basic of the formula.

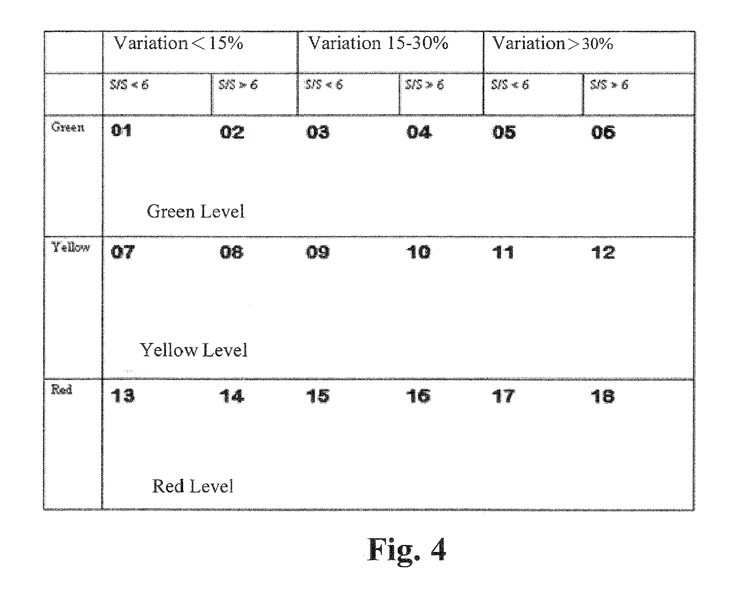

Please refer to Fig. 4. According to the different values of the parameters for monitoring asthmatic patients including PEFR, variation of PEFR and subjective symptom scoring we divided the severity of asthma into 18 categories which are then grouped into 3 color levels such as RED Level, YELLOW Level, and GREEN Level. If the PEFR measured by the patient arrives or above 80% of the best PEFR set by the doctor, it would fall in the GREEN Level. If it arrives 60%-80% of the best PEFR, it would fall in the YELLOW Level. If it is below 60% of the best PEFR, it would fall in the RED Level.

When the patient measure his/her PEFR and enter it into the survey 100 as shown in the Fig. 3, the formula in the mobile phone 2 or the Internet platform 1 can calculate the variation (V) as shown in Fig. 4, which accords:

$$V = \frac{(PEFR\ measured\ in\ the\ afternoon - PEFR\ measured\ in\ the\ morning)}{(PEFR\ measured\ in\ the\ afternoon + PEFR\ measured\ in\ the\ morning)/2}\ 100\%$$

Then we divided each color level into 3 groups according to the value of variation of PEFR ( for example below 15%; between 15 to 30%;and above 30%).

The self-accessing score (S/S) shown in Fig. 4 are based on the subjective symptoms entered by the patient using the survey 100 shown in Fig. 3. There are four questions to be answered by the patient in the survey 100 of Fig. 3 for self-accessing score. Every question can be answered with a number between 0-3. The self-accessing score (S/S) shown in Fig. 4 are the sum total of the four questions. In average, the self-accessing score (S/S) is set as ≥6 or <6, as shown in Fig. 4.

For example, referring to Fig. 4, when the patient is falling to the YELLOW Level according to his best PEFR (i.e. the PEFR measured by the patient is 60%-80% of the best PEFR), if the variation (V) based on the measured PEFR is smaller than 15%, and if his self-assessing score (S/S) is ≥6, then it falls on the YELLOW Level 10.

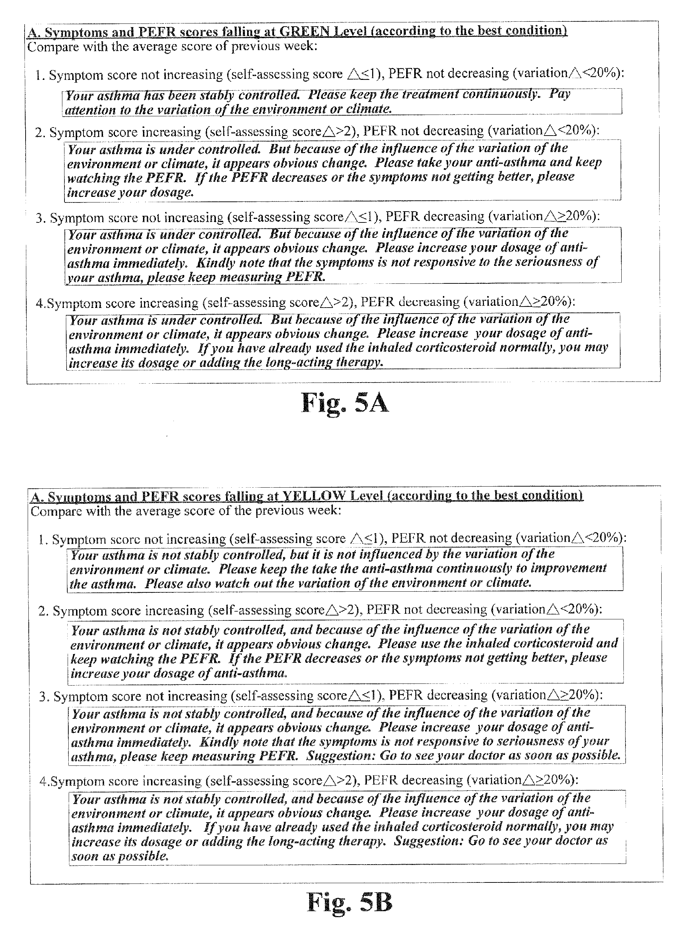

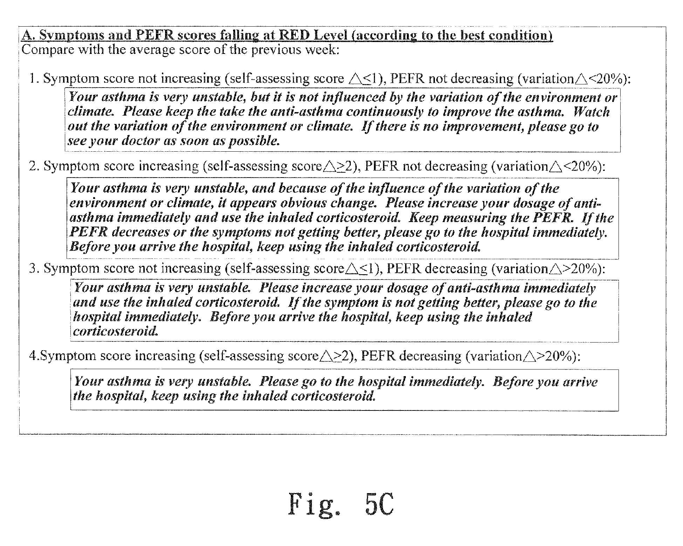

According to the present invention, it will ask patients with asthma to input their physical state information everyday for the regular monitoring. Furthermore, when these patients suffer from short of breath, chest discomfort, persistent cough, and any symptoms implying exacerbation of asthma, they can measure their PEFR and score the asthmatic symptoms; for example, please refer to Fig. 5A-5C. If the physical state information provided by the patient is falling on the GREEN Level (according to the self-assessing scores and the PEFR of the patient), and if the self-assessing scores and the PEFR measured by the patient comparing with the same in previous week show difference of self-assessing score Δ≤1 and variation (V) Δ≥20%, the analyzed information to be informed for the patient is:

"Your asthma is under controlled. But because of the influence of the variation of the environment or climate, it appears obvious change. Please increase your dosage of anti-asthma immediately. Kindly note that the symptoms is not responsive to the seriousness of your asthma, please keep measuring PEFR". As described above, this analyzed information can be calculated directly by the mobile phone 2 or by the computer 1.

In S11, when the physical state information is sent to the Internet platform 1, then in block S15, the Internet platform 1 sends a second message to the mobile phone 2 with the analyzed information as shown in Fig. 5A-5c according to the analysis described as above. Moreover, the analyzed information can be calculated directly by the mobile phone 2 or by sending the second message from the Internet platform 1 to the mobile phone 2 to achieve the informing actively to the patient for his/her health state analysis and management.

Alternatively, in S11, if the physical state information is sent to the Internet platform 1 via Internet using computer 3 or mobile phone 2, the analyzed information can be displayed directly on the computer 3 or the mobile phone 2 with webpage (s) via the same Internet.

In addition, please referring back to the block S113 of Fig. 2: storing the physical state information. The physical state information can be stored, for example, in a record database. Then next, block S115: calculating all physical state information of the patient. For example, the calculation can be a weekly history, a monthly history, or an annually history of the patient. Such histories are advantageously for the hospitals 4 or the doctor 5 as shown in Fig. 1. Especially, the physical state information can make the doctor 5 much easier to monitor the health state of the patient. For example, the doctor 5 would understand that what kind of the environment factor may influence the patient, or how the medicines affect/effect the patient, etc.

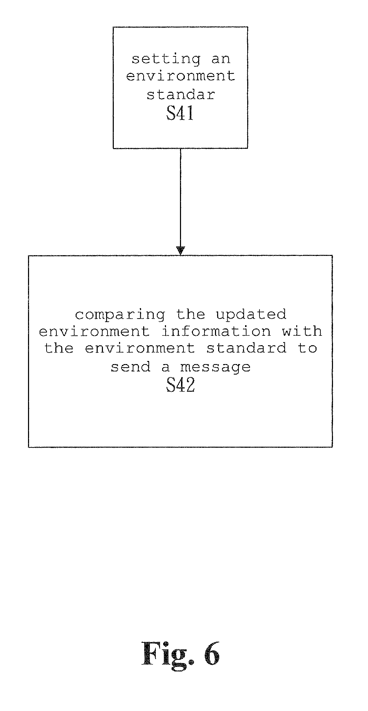

In an embodiment of the present invention, please referring to Fig. 6, because the environment database 13 can be updated with the environment information therein, it may comprise a further step S41: setting an environment standard, such as for specific standard of temperature, moisture, or suspended particulates. And next, in block S42 processes with:

comparing the updated environment information with the environment standard to send a third message. That is, if the updated environment information appears a big weather change, such as temperature decreasing dramatically or dust windstorm is coming, the Internet platform 1 may further actively send the third message to the patient for alerting.

Please referring to Fig. 7, the present invention can use communication between the mobile phone 2 and the Internet platform 1 to achieve the method as described in above. The mobile phone 2 comprises a processor 21 and a memory element 22 containing software 221 therein. The software can be executed to achieve the method as described in above. Furthermore, the mobile phone 2 can be used for displaying the analyzed information as show in Fig. 5A-5C.

Hospital 4 and doctor 5 shown in Fig. 1, and even the patient himself may take advantages from the physical state information and/or the environment information. Thus, the patient may understand his/her own health state based on the history of physical state information. Hospital 4 or doctor 5 may understand what kind of the treatment would be more proper according to the history of physical state information. Furthermore, the hospital 4 can use nursing staff to provide more proper care for the patient and to monitor the patient for more health state, instead of doctors, which means that the medical resources can be reduced accordingly.

Those skilled in the art will readily observe that numerous modifications and alterations of the device and method may be made while retaining the teachings of the invention. Accordingly, the above disclosure should be construed as limited only by the metes and bounds of the appended claims.

**Claims**

1.  A method for analyzing and managing for a health state of a patient comprising:

    receiving at least one physical state information of the patient;
    determining present location information of the patient;
    obtaining environment information associated with the present location information; and
    analyzing the physical state information according to the environment information to generate at least one analyzed information.

2.  The method of Claim 1, wherein the physical state information comprises at least one of sleeping condition data, breathing status level data, coughing status level data, activity status data, and peak expiratory flow rate data.

3.  The method of Claim 1, wherein the physical state information is contained in a first message sent by a mobile phone.

4.  The method of Claim 3, further comprising:

    sending at least one second message to the mobile phone according to the at least one analyzed information.

5.  The method of Claim 1, wherein the environment information comprises at least one of temperature data, moisture data, and suspended particulates data.

6.  The method of Claim 5, wherein the environment information is stored in an environment database, and the environment database is real-time updated for the environment information.

7.  The method of Claim 5, further comprising:

    setting an environment standard; and
    comparing the updated environment information with the environment standard to send a third message.

8.  The method of Claim 1, wherein the at least one physical state information is compared with at least one health standard data stored in a health standard database.

9.  An Internet platform for analyzing and managing for a health state of a patient comprising:

    a processor; and
    a memory element including a software, the memory element electronically connecting with the processor;
    wherein the processor executes the software stored in the memory element to perform:

    receiving at least one physical state information of the patient;
    determining present location information of the patient;
    obtaining environment information associated with the present location information; and
    analyzing the physical state information according to the environment information to generate at least one analyzed information.

10. A mobile phone, comprising:

    a processor; and
    a memory element including a software, the memory element electronically connecting with the processor;
    wherein the processor executes the software stored in the memory element to perform:

    acquiring environment information according to present location information of the patient;
    receiving at least one physical state information of the patient;
    analyzing the physical state information according to the environment information to generate at least one analyzed information.

**Fig. 1**

```
                                  ┌─────────┐
                                  │  Start  │
                                  └─────────┘
                                       │
                                       ▼
┌──────────────────┐        ┌──────────────────────┐
│   storing the    │        │  receiving at least  │
│ physical state   │◄───────│ one physical state   │
│   information    │        │  information of a    │
│      S113        │        │      patient         │
│                  │        │        S11           │
└──────────────────┘        └──────────────────────┘
         │                             │
         ▼                             ▼
┌──────────────────┐        ┌──────────────────────┐
│   calculating    │        │    determining a     │
│  the physical    │        │   present location   │
│     state        │        │  information of the  │
│  information     │        │      patient         │
│      S115        │        │        S12           │
└──────────────────┘        └──────────────────────┘
                                       │
                                       ▼
                            ┌──────────────────────┐
                            │     obtaining an     │
                            │     environment      │
                            │ information based on │
                            │ the present location │
                            │     information      │
                            │         S13          │
                            └──────────────────────┘
                                       │
                                       ▼
                    ┌──────────────────────────────────┐
                    │  analyzing the physical state     │
                    │  information according to the     │
                    │  environment information to       │
                    │ generate at least one analyzed    │
                    │          information              │
                    │             S14                   │
                    └──────────────────────────────────┘
                                       │
                                       ▼
                          ┌──────────────────────────┐
                          │  according to the at     │
                          │  least one analyzed      │
                          │ information, sending     │
                          │ at least one message     │
                          │          S15             │
                          └──────────────────────────┘
```

# Fig. 2

EP 1 857 950 A2

100

A. Please enter your PEFR: _____

B. Most of your activities stay 1)ourdoors 2)indoors _____

C. Please assess your symptoms:

☐ Coughing : 0)None 1)Sometimes 2)Frequent 3)All day long

☐ Difficulty breathing : 0)None 1)Light 2)Frequent 3)Severe

☐ Daily activities affected by disease : 0)Not at all 1)Unable to do more activities 2)Having difficulties performing normal daily activities 3)Unable to do anything

☐ In sleep : 0)Not interrupted by disease 1)Lightly interrupted by disease 2)Waken by disease attack during sleep 3)Unable to sleep

# Fig. 3

EP 1 857 950 A2

| | Variation < 15% | | Variation 15-30% | | Variation > 30% | |
|---|---|---|---|---|---|---|
| | S/S < 6 | S/S > 6 | S/S < 6 | S/S > 6 | S/S < 6 | S/S > 6 |
| Green | **01** | **02** | **03** | **04** | **05** | **06** |
| | Green Level | | | | | |
| Yellow | **07** | **08** | **09** | **10** | **11** | **12** |
| | Yellow Level | | | | | |
| Red | **13** | **14** | **15** | **16** | **17** | **18** |
| | Red Level | | | | | |

**Fig. 4**

**A. Symptoms and PEFR scores falling at GREEN Level (according to the best condition)**

Compare with the average score of previous week:

1. Symptom score not increasing (self-assessing score $\triangle \leq 1$), PEFR not decreasing (variation$\triangle < 20\%$):

> *Your asthma has been stably controlled. Please keep the treatment continuously. Pay attention to the variation of the environment or climate.*

2. Symptom score increasing (self-assessing score$\triangle > 2$), PEFR not decreasing (variation$\triangle < 20\%$):

> *Your asthma is under controlled. But because of the influence of the variation of the environment or climate, it appears obvious change. Please take your anti-asthma and keep watching the PEFR. If the PEFR decreases or the symptoms not getting better, please increase your dosage.*

3. Symptom score not increasing (self-assessing score$\triangle \leq 1$), PEFR decreasing (variation$\triangle \geq 20\%$):

> *Your asthma is under controlled. But because of the influence of the variation of the environment or climate, it appears obvious change. Please increase your dosage of anti-asthma immediately. Kindly note that the symptoms is not responsive to the seriousness of your asthma, please keep measuring PEFR.*

4. Symptom score increasing (self-assessing score$\triangle > 2$), PEFR decreasing (variation$\triangle \geq 20\%$):

> *Your asthma is under controlled. But because of the influence of the variation of the environment or climate, it appears obvious change. Please increase your dosage of anti-asthma immediately. If you have already used the inhaled corticosteroid normally, you may increase its dosage or adding the long-acting therapy.*

## Fig. 5A

**A. Symptoms and PEFR scores falling at YELLOW Level (according to the best condition)**

Compare with the average score of the previous week:

1. Symptom score not increasing (self-assessing score $\triangle \leq 1$), PEFR not decreasing (variation$\triangle < 20\%$):

> *Your asthma is not stably controlled, but it is not influenced by the variation of the environment or climate. Please keep the take the anti-asthma continuously to improvement the asthma. Please also watch out the variation of the environment or climate.*

2. Symptom score increasing (self-assessing score$\triangle > 2$), PEFR not decreasing (variation$\triangle < 20\%$):

> *Your asthma is not stably controlled, and because of the influence of the variation of the environment or climate, it appears obvious change. Please use the inhaled corticosteroid and keep watching the PEFR. If the PEFR decreases or the symptoms not getting better, please increase your dosage of anti-asthma.*

3. Symptom score not increasing (self-assessing score$\triangle \leq 1$), PEFR decreasing (variation$\triangle \geq 20\%$):

> *Your asthma is not stably controlled, and because of the influence of the variation of the environment or climate, it appears obvious change. Please increase your dosage of anti-asthma immediately. Kindly note that the symptoms is not responsive to seriousness of your asthma, please keep measuring PEFR. Suggestion: Go to see your doctor as soon as possible.*

4. Symptom score increasing (self-assessing score$\triangle > 2$), PEFR decreasing (variation$\triangle \geq 20\%$):

> *Your asthma is not stably controlled, and because of the influence of the variation of the environment or climate, it appears obvious change. Please increase your dosage of anti-asthma immediately. If you have already used the inhaled corticosteroid normally, you may increase its dosage or adding the long-acting therapy. Suggestion: Go to see your doctor as soon as possible.*

## Fig. 5B

**A. Symptoms and PEFR scores falling at RED Level (according to the best condition)**
Compare with the average score of the previous week:

1. Symptom score not increasing (self-assessing score $\triangle \leq 1$), PEFR not decreasing (variation$\triangle < 20\%$):

> *Your asthma is very unstable, but it is not influenced by the variation of the environment or climate. Please keep the take the anti-asthma continuously to improve the asthma. Watch out the variation of the environment or climate. If there is no improvement, please go to see your doctor as soon as possible.*

2. Symptom score increasing (self-assessing score$\triangle \geq 2$), PEFR not decreasing (variation$\triangle < 20\%$):

> *Your asthma is very unstable, and because of the influence of the variation of the environment or climate, it appears obvious change. Please increase your dosage of anti-asthma immediately and use the inhaled corticosteroid. Keep measuring the PEFR. If the PEFR decreases or the symptoms not getting better, please go to the hospital immediately. Before you arrive the hospital, keep using the inhaled corticosteroid.*

3. Symptom score not increasing (self-assessing score$\triangle \leq 1$), PEFR decreasing (variation$\triangle > 20\%$):

> *Your asthma is very unstable. Please increase your dosage of anti-asthma immediately and use the inhaled corticosteroid. If the symptom is not getting better, please go to the hospital immediately. Before you arrive the hospital, keep using the inhaled corticosteroid.*

4. Symptom score increasing (self-assessing score$\triangle \geq 2$), PEFR decreasing (variation$\triangle > 20\%$):

> *Your asthma is very unstable. Please go to the hospital immediately. Before you arrive the hospital, keep using the inhaled corticosteroid.*

# Fig. 5C

```
       ┌──────────────────┐
       │  setting an      │
       │  environment     │
       │  standar         │
       │  S41             │
       └──────────────────┘
                 │
                 ▼
┌──────────────────────────────────┐
│                                  │
│  comparing the updated           │
│  environment information with    │
│  the environment standard to     │
│  send a message                  │
│  S42                             │
│                                  │
└──────────────────────────────────┘
```

# Fig. 6

**Fig. 7**